# EUROPÄISCHE PATENTSCHRIFT

(11) **EP 1 302 191 B1**
(45) Veröffentlichungstag und Bekanntmachung des Hinweises auf die Patenterteilung: **26.03.2008**
(21) Anmeldenummer: 02022410.1
(22) Anmeldetag: 04.10.2002
(51) Int. Cl.: A61K 8/04, A61Q 5/06, A61K 8/81

(54) **Aufschäumbare Haarbehandlungsmittel**
Foamable composition for hair care
Composition moussante pour le soin de la chevelure

(30) Priorität: 10.10.2001 DE 10150049
(43) Veröffentlichungstag der Anmeldung: 16.04.2003
(73) Patentinhaber: Henkel Kommanditgesellschaft auf Aktien, 40589 Düsseldorf (DE)
(72) Erfinder: Emmerling, Winfried, Dr., 25436 Tornesch (DE); Richters, Bernd, 21129 Hamburg (DE)

(56) Entgegenhaltungen:
- EP-A- 1 075 832
- EP-A- 1 090 633
- WO-A-01/19946
- WO-A-01/76543
- WO-A-01/91705
- DE-A- 4 435 386
- US-A- 5 833 968
- US-A1- 2001 026 791

## Beschreibung

Die Erfindung betrifft ein Haarbehandlungsmittel, welches als nichtaerosol-Schaum auf das Haar aufgetragen wird, ein Verfahren zur Verschäumung einer gelförmigen kosmetischen Zusammensetzung und die Verwendung der aufgeschäumten kosmetischen Zusammensetzung zur temporären Verformung des Haars sowie ein entsprechendes Verfahren zur temporären Haarverformung.

Keratinische Fasern, insbesondere menschliche Haare, werden heutzutage einer Vielzahl von Behandlungen unterzogen. Dabei spielen die Behandlungen, die zu einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Haarsprays enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Die Druckgasbehälter, mit deren Hilfe ein Produkt durch den Gasdruck des Behälters über ein Ventil verteilt wird, bezeichnet man definitionsgemäß als "Aerosole" (siehe K. Schrader, Grundlagen und Rezepturen der Kosmetika, Dr. Alfred Hüthig Verlag, Heidelberg, 1989.). Als "Nichtaerosol" wird im Umkehrschluß zur Aerosoldefinition eine Packung unter Normaldruck definiert, mit deren Hilfe ein Produkt mittels mechanischer Einwirkung durch ein Pumpsystem verteilt wird. Die Handhabung der Sprüh- oder Pumpbehälter ist leicht und sauber. In der Regel wird sowohl durch ein Aerosol-Spray als auch durch ein Pumpspray eine zufriedenstellend gleichmäßige Verteilung des Polymers auf dem Haar erzielt.

Eine weitere Möglichkeit formgebende Haarbehandlungsmittel zu applizieren ist der Schaum. Der voluminöse Schaum, generiert durch das Treibgas oder mittels eines mechanischen Pumpschaumsystems, wird mit den Händen im Haar verteilt.

Für die Anwendung von Haargelen werden diese Produkte zuerst in der Hand verrieben und dann auf dem Haar verteilt. Die auf dem Markt befindlichen Haargele mit unterschiedlichen Viskositätsbereichen können die Wünsche der Anwender hinsichtlich einfacher Applikation und leichter Verteilung auf dem Haar noch nicht vollständig befriedigen. So ist eine sehr gleichmäßige Verteilung des Produktes auf dem Haar nur schwer zu erreichen und erfordert einen hohen Zeitaufwand. Trotz dieses Nachteils ist ein Produkt in viskoser Gelform erwünscht. Der Anblick eines durchsichtigen viskosen Gels in einem transparenten Behältnis ist ein ästhetischer Genuß und von besonderem Reiz.

Aufgrund dessen besteht ein starkes Interesse an neuen Applikationsformen für Haargele, die die oben genannten Nachteile der Gelform beheben ohne dabei auf die erwünschten Eigenschaften verzichten zu müssen. Aus der EP-B1-0 664 692 sind Gele bekannt, die frei von aliphatischen C₁-C₄-Alkoholen sind und als sogenannte Sprühgele mit handelsüblichen Sprühpumpen versprüht werden können.

Als alternative und verbesserte Applikationsform für Gele kommt neben dem Spray auch der Schaum in Frage. Die dem Fachmann bekannten Gelschäume werden bisher ausschließlich durch Treibgase generiert. Der Einsatz von unter Druck stehenden Treibgasen als Verschäumhilfe ist nach dem Stand der Technik unerläßlich. Ein Grund dafür ist, daß die üblich in Gelen verwendeten Verdickungsmittel auf Basis der Polyacrylate wie z.B. Carbomer^{®} die Schaumbildung erschweren. Die Verwendung von Treibgasen wie FCKW, oder den brennbaren niederen Alkanen bei der Darstellung eines aerosol-Schaumes ist z.B. aus Gründen des Umweltschutzes unerwünscht.

Der Verzicht auf Treibmittel könnte durch die Verwendung eines mechanischen Pumpschaumsystems bewerkstelligt werden. Allerdings können die herkömmlichen viskosen Gele bisher nicht mit mechanischen Pumpschaumsystemen zu einem nichtaerosol-Schaum verschäumt werden. Die Schaumbildung wird zudem durch üblich verwendete Verdickungmittel auf Basis von Polyacrylaten beeinträchtigt. Dem Fachmann ist daher kein nichtaerosol-Schaum aus Gelen bekannt.

Es wurde nun überraschenderweise gefunden, daß sich gelförmige Zubereitungen, mit Hilfe von Luft aus der Umgebung befriedigend zu einem nichtaerosol-Schaum verschäumen lassen, wenn sie unter anderem ein pseudokationisches Verdickungsmittel enthalten.

Gegenstand der vorliegenden Erfindung sind daher Haarbehandlungsmittel, die in Form eines nichtaerosol-Schaums auf das Haar appliziert werden, enthaltend
(A) eine kosmetische Zusammensetzung mit einem pH-Wert kleiner 7, enthaltend mindestens ein pseudokationisches Polymer, mindestens ein Tensid und Wasser , wobei das pseudokationische Polymer ein Copolymerisat aus (i) einer Acrylatkomponente, (ii) einer Aminoacrylatkomponente und (iii) einer Verbindung gemäß Formel (I) ist, 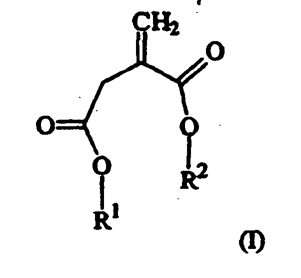 worin
   R¹ und R² unabhängig voneinander stehen für eine C₁-C₃₀-Alkylgruppe oder für eine Gruppe -[(CH₂CH₂O)ₓ-R³], worin R³ steht für Wasserstoff oder eine C₁-C₃₀-Alkylgruppe und x steht für eine ganze Zahl von 1 bis 30, und
(B) Luft als Gaskomponente.

Unter die Stoffklasse der pseudokationischen Polymere fallen Polymere, die üblicherweise eine Aminogruppe enthalten, die bei' bestimmten pH-Werten als quartäre Ammoniumgruppe und somit kationisch vorliegt.

Die Zusammensetzung (A) ist mit einem pH-Wert von pH kleiner 7 sauer eingestellt. Bevorzugt liegt der pH-Wert der Zusammensetzung (A) in einem Bereich von pH 3 bis 6.

Bevorzugt handelt es sich bei der Zusammensetzung (A) um ein Gel. Eine bevorzugte Variante dieser Ausführungsform der Zusammensetzung (A) sind transparente Gele.

Die Verschäumung der Zusammensetzung (A) unter Zuhilfenahme von Luft zu einem nichtaerosol-Schaum wird bevorzugt durch ein mechanisches Pumpschaumsystem bewerkstelligt. Es resultiert daraus ein stabiler Schaum, der sich ausgezeichnet auf dem Haar verteilen läßt und sich für den Anwender angenehm auf der Hand anfühlt. In einer besonders bevorzugten Ausführungsform wird zur Verschäumung das Pumpschaumsystem Airfoamer^{®} (Firma Airspray International) verwendet.

Die Zusammensetzung (A) enthält als Verdickungsmittel zwingend mindestens ein zuvor definiertes pseudokationisches Polymer als Verdickungsmittel.

Besonders bevorzugt ist das pseudokationische Copolymer aus (i) einer Acrylatkomponente, (ii) einer Aminoacrylatkomponente und (iii) C₁₀-C₃₀-Alkyl-PEG-20-Itaconat gemäß Formel (I), wobei
- die Acrylatkomponente (i) aus Acrylsäure, C₁-C₆-Alkylacrylsäureester und C₁-C₆-Alkylmethacrylsäureester und
- die Aminoacrylatkomponente (ii) aus Acrylamid, Methacrylamid, Mono-(C₁-C₄)-alkylamino(C₁-C₄)-alkylacrylat, Di-(C₁-C₄)-alkylamino(C₁-C₄)-alkylacrylat, Mono-(C₁-C₄)-alkylamino(C₁-C₄)-alkylmethacrylat und Di-(C₁-C₄)-alkylamino-(C₁-C₄)-alkylmethacrylat ausgewählt ist.

Ganz besonders bevorzugt ist das unter der INCI-Bezeichung "Acrylates/Aminoacrylates/C10-30 Alkyl PEG-20 Itaconate Copolymer" bekannte Copolymer, das unter dem Namen Structure Plus^{®} von der Firma National Starch & Chemical Ltd. vermarktet wird.

Das pseudokationsiche Polymer ist bevorzugt in einer Menge von 1 bis 2 Gew.%, bezogen auf die Zusammensetzung (A), enthalten. Es ist im Rahmen der Erfindung besonders bevorzugt, 5 bis 10 Gew.%, bezogen auf die Zusammensetzung (A), einer 20 %igen Lösung des pseudokationischen Polymers, einzusetzen.

Desweiteren enthält das erfindungsgemäße Haarbehandlungsmittel zwingend mindestens ein Tensid als oberflächenaktive Verbindung. Als Tenside kommen prinzipiell sowohl anionische als auch ampholytische, zwitterionische, kationische und nichtionische oberflächenaktive Verbindungen in Frage, die für die Verwendung am menschlichen Körper geeignet sind. In einer bevorzugten Ausführungsform enthält die kosmetische Zusammensetzung mindestens ein Tensid ausgewählt aus nichtionischen oberflächenaktiven Verbindungen und kationichen oberflächenaktiven Verbindungen. Kationische oberflächenaktive Verbindungen sind besonders bevorzugt eingesetzte Tenside.

In einer weiteren Ausführungsform enthält die kosmetische Zusammensetzung (A) zusätzlich mindestens ein weiteres Tensid, ausgewählt aus der Gruppe der anionischen und ampholytischen Tenside.

Anionische oberflächenaktive Verbindungen sind gekennzeichnet durch eine wasserlöslich machende anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Beispiele für geeignete anionische oberflächenaktive Verbindungen sind, jeweils in Form der Natrium-, Kalium-, Magnesium- und Ammonium- sowie der Mono-, Di- und Trialkanolammoniumsalze mit 2 oder 3 C-Atomen in der Alkanolgruppe, lineare Fettsäuren (Seifen), Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist, Amidethercarboxylate der Formel [R-NH(-CH₂-CH₂-O)ₙ-CH₂-COO]ₘZ, in der R für einen linearen oder verzweigten, gesättigten oder ungesättigten Acylrest mit 2 bis 29 C-Atomen, n für ganze Zahlen von 1 bis 10, m für die Zahlen 1 oder 2 und Z für ein Kation aus der Gruppe der Alkali- oder Erdalkalimetalle steht, Acylsarcoside, Acyltauride, Acylisothionate, Sulfobernsteinsäuremono- und dialkylester, Sulfobernsteinsäuremonoalkylpolyoxyethylester, lineare Alkansulfonate, lineare Alpha-Olefinsulfonate, Alpha-Sulfofettsäuremethylester, Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist, Gemische oberflächenaktiver Hydroxysulfonate, sulfatierte Hydroxyalkylpolyethylen- und/oder Hydroxyalkylenpropylenglykolether, Sulfonate ungesättigter Fettsäuren, Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen, Kokosmonoglyceridsulfate, Phosphorsäuremono-, -di- und -triester von alkoxylierten Fettalkoholen und ihre Mischungen, wie beispielsweise die unter dem Warenzeichen Hostaphat^{®} vertriebenen Produkte, sowie Ester von hydroxysubstituierten Bi- oder Tricarbonsäuren mit polyhydroxylierten organischen Verbindungen, die aus der Gruppe, die veretherte (C₆-C₁₈)-Alkyl-Polysaccharide mit 1 bis 6 monomeren Saccharideinheiten und veretherte aliphatische (C₆-C₁₆)-Hydroxyalkyl-Polyole mit 2 bis 16 Hydroxylresten umfaßt, ausgewählt sind und die in der Europäischen Patentschrift EP-B1-0 258 814, auf die ausdrücklich Bezug genommen wird, offenbart sind.

Bevorzugte anionische oberflächenaktive Verbindungen sind die Salze der Ethercarbonsäuren sowie phosphatgruppenhaltige Verbindungen, insbesondere die Phosphorsäuremono-, -di- und -triester von ethoxylierten C₁₀-C₁₈-, insbesondere C₁₂-C₁₄-, Fettalkoholen mit Ethoxyliertungsgraden von 2 bis 10, insbesondere von 3 bis 5.

Nichtionische oberflächenaktive Verbindungen enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionogene oberflächenaktive Verbindungen sind die Anlagerungsprodukte von Alkylenoxid, insbesondere Ethylenoxid und Propylenoxid, an Fettalkohole und Fettsäuren, wie beispielsweise PPG-PEG-Alkylether, wie Procetyl AWS^{®} oder PEG-Hydrogenated Castoroil, wie z.B. Cremophor RH 40^{®}.

Als zwitterionische oberflächenaktive Verbindungen werden solche Substanzen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen oberflächenaktiven Verbindungen werden solche Substanzen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Bevorzugte ampholytische Tenside sind das N-Kokosalkylaminopropionat, das Kokosacylaminoethylaminopropionat und das C₁₂₋₁₈-Acylsarcosin.

Beispiele für kationische oberflächenaktive Verbindungen sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Cetyltrimethylammoniumbromid, Stearyltrimethylammoniumchlorid, Distearyldimethylammoniumchlorid, Lauryldimethylammoniumchlorid und Lauryldimethylbenzylammoniumchlorid. Weiterhin können die sehr gut biologisch abbaubaren quaternären Esterverbindungen, wie sie beispielsweise unter dem Warenzeichen Dehyquart^{®}-Reihe vertrieben werden, sowie quaternisierte Proteinhydrolysate und Silikonverbindungen erfindungsgemäß verwendet werden.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden.

Die erfindungsgemäßen Zubereitungen enthalten die Tenside in Mengen von 0,1 bis 10 Gew.-%, bezogen auf die gesamte Zubereitung. Mengen von 0,5 bis 5, insbesondere von 0,6 bis 3 Gew.-% sind bevorzugt.

In einer ersten Ausführungsform enthält das erfindungsgemäße Haarbehandlungmittel, lediglich ein wie oben definiertes pseudokationisches Polymer, welches als pseudokationisches Polymer sowohl als Verdickungsmittel fungiert als auch die Funktion eines Filmbildners zur Formfixierung der Keratinfaser übernimmt, wenn das Haarbehandlungsmittel als Stylingpräparat zum Einsatz kommt.

In einer zweiten Ausführungsform wird der kosmetischen Zusammensetzung (A) mindestens ein weiteres Polymer zugesetzt. Diese weiteren Polymere sind ausgewählt aus
- nichtionischen Polymeren und
- kationischen Polymeren.

Bevorzugte kationische Polymere im Sinne der Erfindung sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Dow Corning^{®} 939 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®} 100 (poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905, HM 552, Hold angeboten werden.
- quaternierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

In einer bevorzugten Ausführungsform werden als zusätzliche Polymere sogenannte Filmbildner, wie das von der Firma BASF unter dem Handelsnamen Luviquat Hold^{®} vertriebene quaternierte Vinylcaprolactam-Vinylpyrrolidon-Vinylymidazol-Copolymer und die unter der Bezeichnung Gafquat^{®} von der Firma GAF vermarkteten Copolymerisate mit der INCI-Bezeichnung Polyquaternium 11 und Polyquaternium 28 verwendet.

Geeignete nichtionische Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Vinylcaprolactam/Vinylpyrrolidon/Dimethylaminoethylmethacrylat-Terpolymere wie sie beispielsweise unter dem Warenzeichen Advantage^{®} LC-E (ISP) vertrieben werden,
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack
- Polyvinylpyrrolidon Polymerisate, wie sie beispielsweise unter der Bezeichnung Luviskol^{®} (BASF) vertrieben werden.
- Glycosidisch substituierte Silicone gemäß der EP-B1-0 612 759.
   Besonders bevorzugt werden die wasserlöslichen Polyvinylpyrrolidon/Vinylacetat-Copolymere, die unter der Bezeichnung Luviskol^{®} von der Firma BASF vertrieben werden sowie das von der Firma ISP unter der Bezeichung Advantage^{®} LC-E vertriebene Vinylcaprolactam/Vinylpyrrolidon/Dimethylaminoethylmethacrylat - Terpolymer verwendet.
   Die erfindungsgemäße Auswahl der Polymer/pseudokationisches Polymer-Kombination (bzw. Filmbildner/Verdicker-Kombination), verbessert die Schaumparameter Stabilität, Volumen und Porigkeit des nichtaerosol-Schaumes und ermöglicht eine sehr gute Schaumausbildung. In einer speziellen Ausführungsform ist die Kombination derart aufeinander abgestimmt, daß die nichtionischen bzw. kationischen filmbildenden Polymere nicht mit dem Verdickungsmittel unter Bildung eines unlöslichen Feststoffs assoziieren, um auf diese Weise für eine sehr gute Verschäumung des Gels zu einem nichtaerosol-Schaum zu gelangen.
   Üblicherweise enthalten die erfindungsgemäßen Zubereitungen jedoch noch weitere Komponenten. Solche weiteren Komponenten sind insbesondere
- Parfümöle
- natürliche Öle, insbesondere pflanzlicher Herkunft, sowie Pflanzenextrakte
- Hydroxycarbonsäuren
- Vitamine
- lösliche UV-Filter
- Farbstoffe zum Anfärben der Zubereitung
- direktziehende Farbstoffe
- Konservierungsmittel
- Silikonöle
- Proteinhydrolysate und/oder Aminosäuren.
   Bevorzugt werden die wasserlöslichen Vertreter dieser Komponenten in der erfindungsgemäßen Zusammensetzung (A) verwendet. Wenn wasserunlösliche Komponenten der oben genannten Liste zum Einsatz kommen, ist die Menge so zu dosieren, daß die Zusammensetzung (A) weiterhin eine homogene Phase bildet.
   Als **natürliche Öle** werden üblicherweise Triglyceride und Mischungen von Triglyceriden eingesetzt .Bevorzugte natürliche Öle sind Kukuinußöl, (süßes) Mandelöl, Walnußöl, Pfirsichkernöl, Avocadoöl, Teebaumöl (Tea tree oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianußöl, Traubenkernöl, Aprikosenkernöl, Babassuöl, Olivenöl, Weizenkeimöl, Kürbiskernöl, Malvenöl, Haselnußöl, Safloröl, Canolaöl, Sasanqua-Öl und Shea-Butter.
   Hinsichtlich der erfindungsgemäß verwendbaren **Pflanzenextrakte** wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.
   Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.
   Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.
   Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.
   Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.
   Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.
   Weiterhin kann es bevorzugt sein, in den erfindungsgemäßen Mitteln Mischungen aus mehreren, insbesondere aus zwei, verschiedenen Pflanzenextrakten einzusetzen.
   Weiterhin ist es erfindungsgemäß bevorzugt, **Hydroxycarbonsäuren** und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.
   Desweiteren kann die erfindungsgemäße kosmetische Zusammensetzung (A) fakultativ **Vitamine** enthalten.
   Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Zubereitung.
   Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal),
- Vitamin C (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.
- Vitamin F. Unter dem Begriff "Vitamin F" werden üblicherweise essentielle Fettsäuren, insbesondere Linolsäure, Linolensäure und Arachidonsäure, verstanden.
- Vitamin H. Als Vitamin H wird die Verbindung (3a*S*,4*S*, 6a*R*)-2-Oxohexa-hydrothienol[3,4-*d*]-imidazol-4-valeriasäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäß verwendeten Zubereitungen Vitamine, Provitamine und Vitaminvorstufen aus den Gruppen A, B, E und H.

Eine weitere fakultative Komponente stellen lösliche **UV-Filter** dar. Löslich im Sinne der Erfindung sind solche UV-Filter, die in den erfindungsgemäßen Zubreitungen in Mengen von mindestens 0,1 Gew.-%, bezogen auf die gesamte Zubereitung, löslich sind. Solche UV-Filter können beispielsweise ausgewählt werden aus substituierten Benzophenonen, p-Aminobenzoesäureestem, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxobom-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 1-(4-tert.-Butylphenyl)-3-(4-methoxyphenyl)-propan-1,3-dion (Butyl methoxydibenzoylmethane; Parsol^{®}1789, Eusolex^{®}9020), ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäureisopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2' ethylhexyl-1'-oxi)-1,3,5-triazin, 3-Imidazol-4-yl-acrylsäureethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6) und 2-Cyano-3,3-diphenylacrylsäure-2'-ethylhexylester. Erfindungsgemäß bevorzugt ist insbesondere Benzophenon-3

Weiterhin sind als konditionierende Wirkstoffe geeignet Silikonöle und Silikon-Gums, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie das Handelsprodukt Fancorsil^{®} LIM-1.

Erfindungsgemäß als konditionierende Wirkstoffe besonders bevorzugt sind kationische **Silikonöle** wie beispielsweise die im Handel erhältlichen Produkte Dow Corning^{®} 939 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Weiterhin können in der erfindungsgemäß verwendeten Zusammensetzung (A) **Proteinhydrolysate und/oder Aminosäuren** und deren Derivate enthalten sein. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgeweicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda) oder Crotein^{®} (Croda) vertrieben.

Die Proteinhydrolysate oder deren Derivate sind in den erfindungsgemäß verwendeten Zubereitungen bevorzugt in Mengen von 0,1 bis 10 Gew.-%, bezogen auf das gesamte Mittel, enthalten. Mengen von 0,1 bis 5 Gew.-% sind besonders bevorzugt.

Bezüglich weiterer üblicher Inhaltsstoffe wird ausdrücklich auf die dem Fachmann bekannten Monographien, beispielsweise K. Schrader, Grundlagen und Rezepturen der Kosmetika, Dr. Alfred Hüthig Verlag, Heidelberg 1989, verwiesen.

Ein zweiter Gegenstand der Erfindung ist die Verwendung einer gelförmigen kosmetischen Zusammensetzung (A) zur Verschäumung zu einem nichtaerosol-Schaum mit der Luft der Umgebung, unter Zuhilfenahme eines mechanischen Pumpschaumsystems.

Ein dritter Gegenstand der Erfindung ist ein Verfahren zur temporären Haarverformung, in dem ein Haarbehandlungsmittel in Form eines nichtaerosol-Schaums, enthaltend
(A) eine kosmetische Zusammensetzung mit einem pH-Wert kleiner 7, enthaltend mindestens ein pseudokationisches Polymer, mindestens ein Tensid und Wasser und, wobei das pseudokationische Polymer ein Copolymerisat aus (i) einer Acrylatkomponente, (ii) einer Aminoacrylatkomponente und (iii) einer Verbindung gemäß Formel (I) ist, 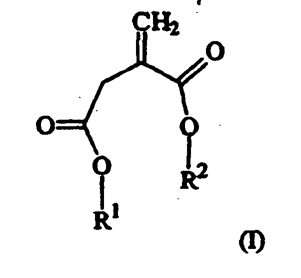 worin
   R¹ und R² unabhängig voneinander stehen für eine C₁-C₃₀-Alkylgruppe oder für eine Gruppe -[(CH₂CH₂O)ₓ-R³], worin R³ steht für Wasserstoff oder eine C₁-C₃₀-Alkylgruppe und x steht für eine ganze Zahl von 1 bis 30, und
(B) Luft als Gaskomponente
bevor und/oder nachdem das Haar nach Wunsch des Anwenders frisiert wird, zur Formfixierung auf das Haar aufgebracht, und das Mittel auf dem Haar belassen wird.

Dabei wird eine handflächengroße Schaummenge des Haarbehandlungsmittels gleichmäßig in dem trockenen oder dem handtuchtrockenen bis nassen Haar verteilt. Als Frisierhilfsmittel vor oder nach der Applikation des Haarbehandlungsmittels eignen sich beispielsweise Kamm, Bürste oder Lockenwickler. Bei der Anwendung an nassem Haar kann anschließend durch Lufttrocknung oder unter Zuhilfenahme eines Föns oder einer Trockenhaube das Haar getrocknet werden.
Ein vierter Gegenstand der Erfindung ist die Verwendung des erfindungsgemäßen Haarbehandlungsmittels zur Haarverformung.

Die nachfolgenden Beispiele sollen den Erfindungsgegenstand näher erläutern.

### Beispiele

### kosmetische Zusammensetzung (A) (erfindungsgemäß)

| | |
|---|---|
| Natriumbenzoat | 0,10 g |
| D-Panthenol 75 L¹ | 0,10 g |
| Allantoin | 0,10 g |
| Milchsäure (80 %ige Lösung) | 0,70 g |
| Luviquat^{®} Hold² | 3,00 g |
| Genamin^{®} CTAC³ | 0,10 g |
| Structure^{®} Plus⁴ | |
| (20 %ige Lösung) | 8,00 g |
| Cremophor^{®} RH 40⁵ | 0,30 g |
| Parfumöl | 0,10 g |
| Wasser (entsalzt) | ad 100 g |

| | |
|---|---|
| ¹ D-Panthenylalkohol, 75 % Aktivsubstanz, (INCI-Bezeichung: Panthenol) (HOFFMANN LA-ROCHE) ² Vinylcaprolactam-Vinylpyrrolidon-Vinylimidazol-quaterniert-Copolymer (INCI-Bezeichung: Polyquaternium 46) (BASF) ³ Trimethylhexadecylammoniumchlorid, 28-30% Aktivsubstanz, (INCI-Bezeichung: Cetrimonium Chloride) (CLARIANT) ⁴ (INCI-Bezeichnung: Acrylates/Aminoacrylates/C10-30 Alkyl PEG-20 Itaconate Copolymer) (NATIONAL STARCH) ⁵ Fettsäurepolyethylenglykolester-Polyethylenglykol-Gemisch (INCI-Bezeichnung: PEG-40 Hydrogenated Castor Oil) (BASF) | |

Natriumbenzoat, D-Panthenol 75 L, Allantoin Genamin^{®} CTAC und Luviquat^{®} Hold werden der Reihe nach unter Rühren in dem Wasser gelöst. Anschließend wird eine Lösung von Cremophor^{®} RH 40 und Parfumöl zugefügt. Es resultiert eine klare Mischung. Zu dieser Mischung wird Structure^{®} Plus gegeben und gerührt, bis sich eine homogene Mischung ergibt. Danach wird mit der Milchsäure der pH Wert auf ca. pH 4 zur Gelbildung eingestellt und die Mischung ruhen gelassen. Die endgültige Konsistenz des Gels bildet sich nach einigen Stunden Standzeit aus.

Das Gel wird in ein Pumpschaumsystem Airfoamer^{®} (Firma Airspray International) gefüllt und mit dessen Hilfe zu einem stabilen Schaum verschäumt, der sich angenehm anfühlt.

## Patentansprüche

1. Haarbehandlungsmittel, die in Form eines nichtaerosol-Schaums auf das Haar appliziert werden, enthaltend
(A) eine gelförmige kosmetische Zusammensetzung mit einem pH-Wert kleiner 7, enthaltend mindestens ein pseudokationisches Polymer, mindestens ein Tensid und Wasser, wobei das pseudokationische Polymer ein Copolymerisat aus (i) einer Acrylatkomponente, (ii) einer Aminoacrylatkomponente und (iii) einer Verbindung gemäß Formel (I) ist, 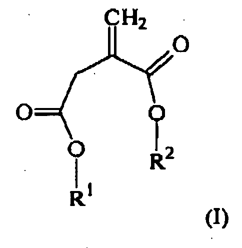 worin
R¹ und R² unabhängig voneinander stehen für eine C₁-C₃₀-Alkylgruppe oder für eine Gruppe -[(CH₂CH₂O)ₓ-R³], worin R³ steht für Wasserstoff oder eine C₁-C₃₀-Alkylgruppe und x steht für eine ganze Zahl von 1 bis 30 und
(B) Luft als Gaskomponente.

2. Haarbehandlungsmittel nach Anspruch 1, **dadurch gekennzeichnet, dass** der Schaum durch ein mechanisches Pumpschaumsystem generiert wird.

3. Haarbehandlungsmittel nach Anspruch 1, **dadurch gekennzeichnet, dass** das pseudokationische Polymer ein Copolymer aus (i) einer Acrylatkomponente, (ii) einer Aminoacrylatkomponente und (iii) C₁₀-C₁₀Alkyl-PEG-20-Itaconat gemäß Formel (I) ist,
**dadurch gekennzeichnet, dass**
- die Acrylatkomponente (i) ausgewählt ist aus Acrylsäure, C₁-C₆-Alkylacrylsäureester und C₁-C₆-Alkylmethaerylsäurcester, und
- die Aminoacrylatkomponente (ii) ausgewählt ist aus Acrylamid, Methacrylamid, Mono-(C₁-C₄)-alkylamino(C₁-C₄)-alkylacrylat, Di-(C₁-C₄)-alkylamino(C₁-C₄)-alkylacrylat Mono-(C₁-C₄)-alkylamino(C₁-C₄)-alkylmethacrylat und Di-(C₁-C₄)-alkylamino-(C₁-C₄)-alkylmethacrylat.

4. Haarbehandlungsmittel nach einem der Ansprüche 1 bis 3, **dadurch gekennzeichnet, dass** das pseudokationische Polymer in einer Menge von 1 bis 2 Gew.% bezogen auf die Masse der Zusammensetzung (A), enthalten sind.

5. Haarbehandlungsmittel nach einem der Ansprüche 1 bis 4, **dadurch gekennzeichnet, dass** die kosmetische Zusammensetzung (A) zusätzlich mindestens ein weiteres Polymer enthält.

6. Haarbehandlungsmittel nach einem der Ansprüche 1 bis 5, **dadurch gekennzeichnet, dass** die kosmetische Zusammensetzung (A) mindestens ein kationisches oder nichtionisches Tensid enthält.

7. Haarbehandlungsmittel nach Anspruch 6, **dadurch gekennzeichnet, dass** das kationische Tensid ausgewählt ist aus quartären Ammoniumverbindungen.

8. Haarbehandlungsmittel nach einem der Ansprüche 6 oder 7, **dadurch gekennzeichnet, dass** die quartären Ammoniumverbindungen ausgewählt sind aus Cetyltrimethylammoniumchlorid, Cetyltrimethylammoniumbromid, Stearylirimethylammoniumchlorid, Distearyldimethylammoniumchlorid, Lauryldimethylammoniumchlorid und Lauryldimethylbenzylammoniumchlorid.

9. Haarbehandlungsmittel nach einem der Ansprüche 1 bis 8, **dadurch gekennzeichnet, dass** das kationische und/oder nichtionische Tensid in einer Menge von 0,5 bis 5, insbesondere von 0,6 bis 3 Gew.-%, bezogen auf die Zusammensetzung (A), enthalten ist.

10. Haarbehandlungsmittel nach einem der Ansprüche 1 bis 9, **dadurch gekennzeichnet, dass** die kosmetische Zusammensetzung (A) zusätzlich mindestens ein weiteres Tensid, ausgewählt aus der Gruppe der anionischen und ampholytischen Tenside enthält.

11. Verwendung einer gelförmigen kosmetischen Zusammensetzung (A), gemäß Anspruch 1, zur Verschäumung zu einem nichtaerosol-Schaum mit der Luft der Umgebung, unter Zuhilfenahme eines mechanischen Pumpschaumsystems.

12. Verfahren zur temporären Haarverformung, **dadurch gekennzeichnet, dass** ein Haarbehandlungsmittel, in Form eines nichtaerosolen Schaums, gemäß einem der Ansprüche 1 bis 10, bevor und/oder nachdem das Haar nach Wunsch des Anwenders frisiert wird, zur Formfixierung auf das Haar aufgebracht, und das Mittel auf dem Haar belassen wird.

13. Verwendung eines Haarbehandlungsmittels nach einem der Ansprüche 1 bis 10 zur Haar-verfünmung.

## Claims

1. Hair treatment compositions which are applied to the hair in the form of a nonaerosol foam, comprising
(A) a gel-like cosmetic composition with a pH of less than 7, comprising at least one pseudocationic polymer, at least one surfactant and water, where the pseudocationic polymer is a copolymer of (i) an acrylate component, (ii) an aminoacrylate component and (iii) a compound according to the formula (I) 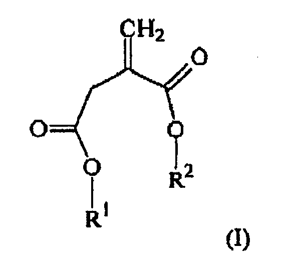 in which
R¹ and R², independently of one another, are a C₁-C₃₀-alkyl group or a group -[(CH₂CH₂O)ₓ-R³], in which R³ is hydrogen or a C₁-C₃₀-alkyl group and x is an integer from 1 to 30 and
(B) air as gas component.

2. Hair treatment compositions according to Claim 1, **characterized in that** the foam is generated by a mechanical pump foam system.

3. Hair treatment compositions according to Claim 1, **characterized in that** the pseudocationic polymer is a copolymer of (i) an acrylate component, (ii) an aminoacrylate component and (iii) C₁₀-C₃₀-alkyl PEG-20 itaconate according to formula (I),
**characterized in that**
- the acrylate component (i) is selected from acrylic acid, C₁-C₆-alkylacrylic acid esters and C₁-C₆-alkylmethacrylic acid esters, and
- the aminoacrylate component (ii) is selected from acrylamide, methacrylamide, mono-(C₁-C₄)-alkyl amino (C₁-C₄) -alkylacrylate, di-(C₁-C₄)-alkyl amino (C₁-C₄)-alkylacrylate, mono-(C₁-C₄)-alkyl amino(C₁-C₄)-alkylmethacrylate and di-(C₁-C₄)-alkyl amino(C₁-C₄)-alkylmethacrylate.

4. Hair treatment compositions according to one of Claims 1 to 3, **characterized in that** the pseudocationic polymer is present in an amount of from 1 to 2% by weight, based on the mass of the composition (A).

5. Hair treatment compositions according to one of Claims 1 to 4, **characterized in that** the cosmetic composition (A) additionally comprises at least one further polymer.

6. Hair treatment compositions according to one of Claims 1 to 5, **characterized in that** the cosmetic composition (A) comprises at least one cationic or nonionic surfactant.

7. Hair treatment compositions according to Claim 6, **characterized in that** the cationic surfactant is selected from quaternary ammonium compounds.

8. Hair treatment compositions according to one of Claims 6 or 7, **characterized in that** the quaternary ammonium compounds are selected from cetyltrimethylammonium chloride, cetyltrimethylammonium bromide, stearyltrimethylammonium chloride, distearyldimethylammonium chloride, lauryldimethylammonium chloride and lauryldimethylbenzylammonium chloride.

9. Hair treatment compositions according to one of Claims 1 to 8, **characterized in that** the cationic and/or nonionic surfactant is present in an amount of from 0.5 to 5% by weight, in particular from 0.6 to 3% by weight, based on the composition (A).

10. Hair treatment compositions according to one of Claims 1 to 9, **characterized in that** the cosmetic composition (A) additionally comprises at least one further surfactant selected from the group of anionic and ampholytic surfactants.

11. Use of a gel-like cosmetic composition (A) according to Claim 1 for foaming to give a nonaerosol foam with the surrounding air, with the help of a mechanical pump foam system.

12. Method of temporarily shaping hair, **characterized in that** a hair treatment composition, in the form of a nonaerosol foam, according to one of Claims 1 to 10, is applied to the hair, before and/or after the hair is styled as desired by the user, for fixing the shape, and the composition is left on the hair.

13. Use of a hair treatment composition according to one of Claims 1 to 10 for shaping hair.

## Revendications

1. Produit de traitement des cheveux que l'on applique sur la chevelure sous la forme d'une mousse qui n'est pas un aérosol, et qui contient :
(A) une composition cosmétique en forme de gel ayant un pH inférieur à 7, contenant au moins un polymère pseudo-cationique, au moins un agent tensio-actif et de l'eau, où le polymère pseudo-cationique est un copolymère constitué de (i) un composant acrylate, (ii) un composant aminoacrylate et (iii) un composé selon la formule (I) 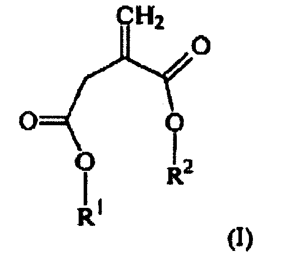 dans laquelle
R¹ et R² représentent indépendamment l'un de l'autre un groupe alkyle en C₁ à C₃₀ ou un groupe
-[(CH₂CH₂O)ₓ-R³], dans lequel R³ représente l'hydrogène ou un groupe alkyle en C₁ à C₃₀, et x représente un nombre entier allant de 1 à 30, et
(B) de l'air comme composant gazeux.

2. Produit de traitement des cheveux selon la revendication 1, **caractérisé en ce que** la mousse est engendrée par un système mécanique de pompage de mousse.

3. Produit de traitement des cheveux selon la revendication 1, **caractérisé en ce que** le polymère pseudo-cationique est un polymère choisi parmi (i) un composant acrylate, (ii) un composant aminoacrylate et (iii) un itaconate d'alkyle en C₁₀ à C₃₀-PEG-20 selon la formule (I), **caractérisé en ce que**
- le composant acrylate (i) est choisi parmi l'acide acrylique, un ester d'acide alkyle en C₁ à C₆-acrylique et un ester d'acide alkyle en C₁ à C₆-méthacrylique, et
- le composant aminoacrylate (ii) est choisi parmi l'acrylamide, le méthacrylamide, le mono-(alkyle en C₁ à C₄)-amino-(alkyle en C₁ à C₄)-acrylate, le di-(alkyle en C₁ à C₄)-amino-(alkyle en C₁ à C₄)-acrylate, le mono-(alkyle en C₁ à C₄)-amino-(alkyle en C₁ à C₄)-méthacrylate et le di-(alkyle en C₁ à C₄)-amino-(alkyle en C₁ à C₄)-méthacrylate.

4. Produit de traitement des cheveux selon l'une des revendications 1 à 3, **caractérisé en ce que** le polymère pseudo-cationique est contenu en une quantité de 1 à 2 % en poids par rapport à la masse de la composition (A).

5. Produit de traitement des cheveux selon l'une des revendication 1 à 4, **caractérisé en ce que** la composition cosmétique (A) contient en outre au moins un autre polymère.

6. Produit de traitement des cheveux selon l'une des revendications 1 à 5, **caractérisé en ce que** la composition cosmétique (A) contient au moins un agent tensioactif cationique ou non ionique.

7. Produit de traitement des cheveux selon la revendication 6, **caractérisé en ce que** l'agent tensioactif cationique est choisi parmi les composés d'ammonium quaternaire.

8. Produit de traitement des cheveux selon l'une des revendications 6 ou 7, **caractérisé en ce que** les composés d'ammonium quaternaire sont choisis parmi le chlorure de cétyltriméthylammonium, le bromure de cétyltriméthylammonium, le chlorure de stéaryltriméthylammonium, le chlorure de distéaryldiméthylammonium, le chlorure de lauryldiméthylammonium et le chlorure de lauryldiméthylbenzylammonium.

9. Produit de traitement des cheveux selon l'une des revendications 1 à 8, **caractérisé en ce que** l'agent tensioactif cationique et/ou non ionique est contenu en une quantité de 0,5 à 5, en particulier de 0,6 à 3 % en poids, par rapport à la composition (A).

10. Produit de traitement des cheveux selon l'une des revendications 1 à 9, **caractérisé en ce que** la composition cosmétique (A) contient en outre au moins un autre agent tensioactif, choisi dans le groupe des agents tensioactifs anioniques et ampholytiques.

11. Utilisation d'une composition cosmétique (A) sous forme de gel, selon la revendication 1, aux fins de moussage sous la forme d'une mousse qui n'est pas un aérosol, avec l'air environnant, à l'aide d'un système mécanique de pompage de mousse.

12. Procédé de mise en forme temporaire de la chevelure, **caractérisé en ce qu'**on dépose sur les cheveux un produit de traitement des cheveux, sous la forme d'une mousse qui n'est pas un aérosol, selon l'une des revendications 1 à 10, avant et/ou après le coiffage des cheveux selon le désir de l'utilisateur, aux fins de fixation de la forme de la chevelure, et **en ce qu'**on laisse le produit sur les cheveux.

13. Utilisation d'un produit de traitement des cheveux selon l'une des revendications 1 à 10 pour la mise en forme de la chevelure.
